# EUROPÄISCHE PATENTANMELDUNG

(11) **EP 3 410 386 A1**
(43) Veröffentlichungstag der Anmeldung: **05.12.2018**
(21) Anmeldenummer: 17173625.9
(22) Anmeldetag: 31.05.2017
(51) Int. Cl.: G06Q 50/24, G06Q 50/22

(54) **VERFAHREN ZUM VERWALTEN VON DATEN IM GESUNDHEITSBEREICH**

(71) Anmelder: Greiner Bio-One GmbH, 4550 Kremsmünster (AT)
(72) Erfinder: RAMPETSREITER, Christoph, 4550 Kremsmünster (AT)
(74) Vertreter: Burger, Hannes

(57) **Zusammenfassung**

Verfahren zur Zusammenführung von ersten Daten (1) und zweiten Daten (2), welche Daten (1,2) erste Attribute beziehungsweise zweite Attribute umfassen und welche Daten über einen ersten Code (4) beziehungsweise über einen zweiten Code (5) auslesbar sind, umfassend die folgenden Verfahrensschritte:
- Abrufen der ersten Daten (1),
- Abrufen der zweiten Daten (2),
- Zusammenführen der ersten Daten (1) und der zweiten Daten (2),
- Generierung von dritten Daten (3) umfassend eine erste Teilmenge (15) der ersten Attribute und eine zweite Teilmenge (16) der zweiten Attribute,
- Generierung eines dritten Codes (6) zum Auslesen der dritten Daten,
- Überschreiben des ersten Codes (4) mit dem dritten Code (6).

## Beschreibung

Die Erfindung betrifft ein Verfahren zum Verwalten von Daten im Gesundheitsbereich.

Die Daten umfassen Attribute und sind über das Lesen eines Codes von einer Datenbank abrufbar. Diese Daten sind mit einem Objekt verknüpft, welches eine von einer Person entnommene Probe umfasst oder darin lagert.

Ein Code kann beispielsweise ein Barcode, ein QR-Code oder ein sonstiger Code nach dem Stand der Technik sein.

WO2011088110 betrifft ein Verfahren zum Verwalten von medizinischen Gütern. In [0042]ff von WO2011088110 ist eine Datenverarbeitungsvorrichtung (data-logging device) erwähnt, welche jedoch nicht die Merkmale zur Durchführung des erfindungsgemäßen Verfahrens aufweist. WO2011088110 hat das der im Folgenden beschriebenen Erfindung zu Grunde liegende Problem des Zusammenführens von Codes nicht.

WO2008042354 ist kein relevanter Stand zu der im Folgenden offenbarten Erfindung.

US20030120633 ist in WO2008042354 als Stand der Technik erwähnt.

EP1838589B1, US2005145048A sind kein relevanter Stand der Technik.

US20160070865A1 (US2012284042) offenbart nicht das Merkmal des Generierens eines dritten Codes aus einem ersten Code und einem zweiten Code. Weiters fehlt in US20160070865A1 ein Hinweis auf das Überschreiben eines Codes.

WO0120532 (US6832722) offenbart auf Seite 6, Zeile 4ff die Assoziierung von Patientendaten, Analysedaten mit einem Code. WO0120532 offenbart nicht das Überschreiben eines ersten Codes. Das Verfahren gemäß WO0120532 ist somit darauf beschränkt, dass nur Maschinen eingesetzt werden können, welche Maschinen diesen Code lesen und verarbeiten können.

WO2006053316 (US7278579) erwähnt in [0003] die Verknüpfung der unterschiedlichen Systeme wie ein Krankenhausinformationssystem (Hospital Information System HIS) und ein Laborinformationssystem (Laboratory Information System LIS). WO2006053316 erwähnt nicht das Überschreiben von Codes beim Wechsel von einem System zu einem anderen System.

Die in WO2006019392 (US8719053) offenbarte Erfindung basiert auf dem Problem der beschränkten Einsetzbarkeit des LIS-Systems hinsichtlich einer Kontrolle im Labor (siehe WO2006019392, Seite 2ff). Die in WO2006019392 offenbarte Lösung basiert auf einer Verknüpfung von Servern. Nachdem WO2006019392 nicht das Problem der Kompatibilität von HIS-Systemen und LIS-Systemen erwähnt, sind in WO2006019392 auch nicht die Merkmale der hier offenbarten Erfindung zu finden. In der Figurenbeschreibung zu den Figuren 25 und 26 (Seite 73, Zeile 25ff) ist in WO2006019392 lediglich erwähnt, dass ein Barcode auf eine Folie aufgeklebt werden kann. Es fehlt hierbei der Hinweis, dass der auf die Folie aufgeklebte Barcode einen bestehenden Code verdecken kann.

WO2008156566A1 (US2008235055) erwähnt ebenso die mangelnde Einsetzbarkeit von LIS, um den Arbeitsfluss zwischen den Geräten zu steuern. Die in WO2008156566A1 aufgezeigte Lösung umfasst unter anderem eine abgeglichene Kennzeichnung (siehe Seite 5). Es findet sich in diesem Dokument kein Hinweis auf das Setzen eines Labels über ein bestehendes Label.

WO2009052501A2 (US8567663) offenbart ebenso nicht das Überkleben eines ersten Codes mit einem zweiten Code.

Die oben angeführten Dokumente liefern keine Lösung zum Verbinden der unterschiedlichen, nicht kompatiblen Systeme wie beispielsweise HIS (Hospital Information System) und LIS (Laboratory Information System). Eine solche Lösung zum Verbinden der beiden Systeme ist in einem Gesundheitsbetrieb notwendig, da die im Labor verwendeten Geräte die im Krankenhaus verwendeten Codes nicht auslesen können. Ebenso ist es nicht möglich, mittels der im Labor verwendeten Geräte die Codes, die mittels der im Krankenhaus verwendeten Geräte erstellt wurden, auszulesen.

Die im Folgenden offenbarte Erfindung stellt sich die Aufgabe, ein einfaches Verfahren zu offenbaren, mit Hilfe dessen die ansonsten nicht kompatiblen Verwaltungssysteme wie beispielsweise ein Krankenhausinformationssystem (Hospital Information System, kurz HIS) und ein Laborinformationssystem (Laboratory Information System, kurz LIS) miteinander kombiniert werden.

Der Umgang mit Patientendaten unterliegt besonderen datenrechtlichen Bestimmungen. Die Erfindung stellt sich weiters die Aufgabe, die Übertragung der Daten zwischen den Verwaltungssystemen wie beispielsweise dem HIS und dem LIS so zu gestalten, dass nur freigegebene Daten des einen Verwaltungssystems wie beispielsweise HIS bei Verwendung des anderen Verwaltungssystems wie beispielsweise LIS ausgelesen werden können.

Diese Aufgabe wird durch ein Verfahren gemäß den Ansprüchen gelöst. Das erfindungsgemäße Verfahren umfasst die folgenden Verfahrensschritte:
- Abrufen der ersten Daten und der zweiten Daten,
- Zusammenführen der ersten Daten und der zweiten Daten,
- Generierung von dritten Daten umfassend eine erste Teilmenge der ersten Attribute und eine zweite Teilmenge der zweiten Attribute,
- Generierung eines dritten Codes zum Auslesen der dritten Daten,
- Überschreiben des ersten Codes mit dem dritten Code.

Das Abrufen der ersten Daten aus einer ersten Datenbank und das Abrufen der zweiten Daten aus einer zweiten Datenbank kann mit dem Lesen eines ersten Codes beziehungsweise eines zweiten Codes verknüpft sein. Durch das Lesen des Codes erhält der Benutzer die Berechtigung zum Abrufen der mit dem Code verknüpften Daten.

Das erfindungsgemäße Verfahren kann sich dadurch auszeichnen, dass der Benutzer nur erste Teilmengen von ersten Attributen der ersten Daten und/oder zweite Teilmengen von zweiten Attributen der zweiten Daten abrufen kann.

Die ersten Daten sind beispielsweise krankenhausbezogene Daten und können im Krankenhaus erstellt werden. Die ersten Daten umfassen erste Attribute wie patientenbezogene Daten wie Name, Geschlecht, Adresse, Krankheitssymptome und Daten über die Entnahme der Probe. Die ersten Attribute können auch Angaben über das Objekt enthalten, in welchem Objekt die Probe gelagert ist. Allenfalls können die ersten Daten eine HIS-Identifikationsnummer der Probe umfassen. Die ersten Daten sind in einer HIS-Datenbank abgespeichert und können nur von Geräten abgerufen werden, die den HIS-kompatiblen und HIS-intern freigegebenen ersten Code lesen können.

Die zweiten Daten können beispielsweise laborbezogene Daten sein. Diese umfassen zweite Attribute zur Durchführung einer Probenuntersuchung im Labor. Die zweiten Attribute können weiters Informationen über die im Labor vorhandenen Gerätschaften und die zeitliche Einteilung umfassen. Allenfalls können die zweiten Daten auch eine LIS-Identifikationsnummer der Probe umfassen. Die zweiten Daten sind auf einer LIS-Datenbank abgespeichert und können nur von Geräten abgerufen werden, die den LIS-kompatiblen oder LIS-intern freigegebenen Code lesen können.

Die Möglichkeit des Auslesens des Codes durch ein Gerät und somit die Kompatibilität von Verwaltungssystemen kann nach dem Stand der Technik durch die Gestaltung des Codes beschränkt sein. Weiters kann der Code mit einem Schutz wie beispielsweise ein Passwort oder ein Sicherheitsschlüssel versehen sein, sodass der Code nicht durch ein beliebiges Gerät auslesbar ist.

Nach dem Zusammenführen von ersten Teilmengen der ersten Attribute der ersten Daten und von zweiten Teilmengen von zweiten Attributen der zweiten Daten und das Generieren eines dritten Codes wird ein für zumindest oder nur das verwendete Verwaltungssystem lesbarer Code erstellt. Der Code kann auch für beide verwendeten Verwaltungssysteme lesbar sein. Der Code kann hierzu auch für das jeweilige Verwaltungssystem lesbare Codeabschnitte umfassen.

Die Offenbarung des erfindungsgemäßen Verfahrens schließt nicht aus, dass eine Teilmenge von Attributen der Daten sämtliche Attribute der jeweiligen Daten umfasst.

Die hier diskutierte Erfindung kann die oben angeführte Problemstellung des Datenschutzes so lösen, dass nur eine erste Teilmenge der ersten Attribute mit einer zweiten Teilmenge der zweiten Attribute der zweiten Daten zusammengeführt werden. Diese Teilmengen können ausschließlich Attribute umfassen, welche zur Untersuchung der Probe unbedingt erforderlich sind.

Die Teilmenge der ersten Attribute kann beispielsweise keine Attribute wie Namen, genaues Geburtsdatum des Patienten umfassen, welche zur Untersuchung der Probe nicht notwendig sind.

Durch das Nichtverknüpfen jener Teilmengen der ersten Attribute, welche ursprünglich in der einen Datenbank abgespeichert sind und nicht für die Untersuchung der Probe unter Verwendung der anderen Datenbank erforderlich sind, können diese auch nicht bei Benutzung des anderen Verwaltungssystems ausgelesen werden. Das erfindungsgemäße Verfahren ist als manipulationssicher anzusehen.

Das Überschreiben des ersten Codes erfolgt unter Anwendung von Methoden nach dem Stand der Technik so, dass der erste Code nach dem Überschreiben nicht mehr lesbar ist. In den nachfolgend beschriebenen Ausführungsformen des erfindungsgemäßen Verfahrens sind beispielhaft Verfahren zum Überschreiben des ersten Codes durch Aufbringen des dritten Codes beschrieben.

Ein Objekt, das zwei Codes wie einen ersten Code und einen dritten Code umfasst, kann bei der Verwaltung des Objektes in einem Verwaltungssystem hinderlich sein, da im Zuge der Analyse der Probe versucht werden kann, den ersten Code anstelle des dritten Codes auszulesen. Neben einem Zeitverlust besteht auch die Gefahr, dass durch das Lesen des falschen Codes falsche Daten aus einer Datenbank abgerufen werden. Das Überschreiben des ersten Codes muss in der Art erfolgen, dass der überschriebene erste Code nicht als Code erfasst oder nicht ausgelesen werden kann.

Der zweite Code und der dritte Code können ident sein. Der zweite Code und der dritte Code können ident gestaltet sein. Der zweite Code und der dritte Code können derselbe Code sein.

Insbesondere im Gesundheitswesen ist sicherzustellen, dass die Probe den richtigen Untersuchungen zugewiesen wird sowie die richtigen Untersuchungen an der Probe durchgeführt werden. Das erfindungsgemäße Verfahren kann hierzu Überprüfungsroutinen umfassen, welche ein Verwechseln der zu untersuchenden Probe vermeiden sollen.

Das erfindungsgemäße Verfahren kann sich dadurch auszeichnen, dass der Verfahrensschritt des Zusammenführens von ersten Daten und zweiten Daten in Abhängigkeit von passenden Attributen der ersten Daten und der zweiten Daten oder von einer Anzahl von passenden Attributen durchgeführt wird. Das erfindungsgemäße Verfahren kann Routinen zum Abgleich der ersten Attribute und der zweiten Attribute umfassen.

Ein erstes Attribut und ein zweites Attribut werden als passende Attribute angesehen, wenn die Attribute den selben Wert wie beispielsweise dieselbe Identifikationsnummer aufweisen.

Die ersten Daten können ein erstes Attribut zur Identifikation und die zweiten Daten ein zweites Attribut zur Identifikation umfassen. Das erfindungsgemäße Verfahren kann einen Abgleich dieser Attribute umfassen, wobei der Verfahrensschritt des Zusammenführens der Daten und des Generierens von dritten Daten in Abhängigkeit davon erfolgt, dass diese Attribute zur Identifikation gleich und sohin passend sind.

Ein erstes Attribut und ein zweites Attribut werden als passende Attribute angesehen, wenn durch das erste Attribut eine Anforderung definiert wird, welche durch das zweite Attribut erfüllt wird.

Es können beispielsweise Attribute betreffend zeitliche Angaben abgeglichen werden. Die zweiten Daten können zweite Attribute umfassen, dass der die Probe beinhaltende Behälter und/oder die im Behälter eingelagerte Probe und/oder das im Behälter eingelagerte Additiv nicht älter als ein gewisses Datum sein darf. Die ersten Daten müssen ein erstes Attribut betreffend das Alter des Behälters und/oder der Probe und/oder des Additivs aufweisen, sodass ein Abgleich dieser ersten Attribute und zweiten Attribute erfolgen kann. Falls ein Abgleich dieser ersten Attribute und dieser zweiten Attribute definierte Kriterien erfüllt und die Attribute passend sind, werden die ersten Daten und die zweiten Daten unter Generierung von dritten Daten zusammengeführt.

Es können weiters laborseitig oder bei Anwendung des erfindungsgemäßen Verfahrens durch ein zweites Attribut Anforderungen an den die Probe beinhaltenden Behälter gestellt werden. Das zweite Attribut kann beispielsweise die Anforderung umfassen, dass der Behälter ein bestimmtes Additiv zur Vermengung mit der Probe umfasst. Durch einen Abgleich der als erstes Attribut abgespeicherten Eigenschaften des Behälters mit den als zweites Attribut abgespeicherten geforderten Eigenschaften des Behälters kann ein abzufragendes Kriterium vor dem Zusammenführen der Daten in das erfindungsgemäße Verfahren aufgenommen werden.

Das erfindungsgemäße Verfahren kann auch Verfahrensschritte einschließen, dass ein mit den ersten Daten verknüpftes erstes Objekt ausgeschieden wird, falls ein erstes Attribut zu einem zugeordneten zweiten Attribut nicht passend ist oder die Anzahl der passenden Attribute kleiner als ein definierter Schwellenwert ist. Üblicher Weise wird der Schwellenwert so definiert, dass alle Attribute passend sein müssen.

Die dritten Daten können einen ersten Zeitpunkt des Auslesens der ersten Daten umfassen. Der erfindungsgemäße Verfahren wird vorzugsweise mit einer geeigneten Vorrichtung durchgeführt. Das Auslesen der ersten Daten ist der erste Zeitpunkt, bei welchem mittels der Vorrichtung das Vorhandensein der Probe in der Vorrichtung registriert wird.

Die Vorrichtung zur Durchführung des erfindungsgemäßen Verfahrens kann neben Merkmalen zur Durchführung des erfindungsgemäßen Verfahrens weitere Merkmale beziehungsweise Funktionen wie das Aufbewahren der Probe bei einer definierten Temperatur umfassen. Insbesondere bei Anwendungen im Gesundheitswesen kann der Zeitpunkt, ab welchem die Probe unter gewissen Bedingungen gelagert wird, für die Haltbarkeit der Probe entscheidend sein.

Das erfindungsgemäße Verfahren kann einen Verfahrensschritt umfassen, dass ein mit den ersten Daten verknüpftes Objekt ausgeschieden wird, wenn der erste Zeitpunkt nach einem definierten Auslesezeitpunkt liegt. Somit wird ein mit den ersten Daten verknüpftes Objekt, welches zu spät in die Vorrichtung eingebracht wird, nicht an das Labor zur weiteren Untersuchung weitergeleitet.

Die hier offenbarte Erfindung betrifft auch eine Vorrichtung zur Durchführung des Verfahrens wie oben beschrieben. Die erfindungsgemäße Vorrichtung umfasst eine erste Datenausleseeinheit zum Auslesen der ersten Daten, eine zweite Datenausleseeinheit zum Auslesen der zweiten Daten, eine Codegenerierungseinheit zum Generieren des dritten Codes und eine Überschreibeinheit zum Überschreiben des ersten Codes durch den dritten Code.

Die Einheiten der Vorrichtung können mit einer Zeiterfassungseinheit gekoppelt sein. Die Zeiterfassungseinheit registriert, zu welchem Zeitpunkt die einzelnen Schritte durchgeführt werden.

Die erste Datenausleseeinheit kann mit der Zeiterfassungseinheit gekoppelt sein, um so den Zeitpunkt des Einbringens des Objektes in die Vorrichtung zu erfassen. Die erste Datenausleseeinheit ist vorzugsweise nahe einer Eingabeeinheit angeordnet, sodass der Zeitpunkt des Auslesens der ersten Daten dem Zeitpunkt der Eingabe des Objektes in die Vorrichtung entspricht. Die Überschreibeinheit kann ebenso mit der Zeiterfassungseinheit gekoppelt sein, um den Zeitpunkt des Überschreibens zu erfassen. Sofern die Vorrichtung keine Ausgabeeinheit umfasst, welche Ausgabeeinheit mit der Zeiterfassung gekoppelt ist, kann der Zeitpunkt des Überschreibens des ersten Codes als ein dem Zeitpunkt des Ausgebens naher Zeitpunkt angesehen werden.

Die Codegenerierungseinheit kann einen Drucker umfassen, mittels welchem Drucker der dritte Code auf eine Folie ausgedruckt wird, und
die Überschreibeinheit die Folie den ersten Code verdeckend am Objekt anbringt.

Zum besseren Verständnis der Erfindung wird diese anhand der nachfolgenden Figuren näher erläutert.

Figur 1 bis Figur 4 zeigen eine Ausführungsform des erfindungsgemäßen Verfahrens.

Figur 5 zeigt eine Ausführungsform der erfindungsgemäßen Vorrichtung zur Durchführung des oben beschriebenen Verfahrens.

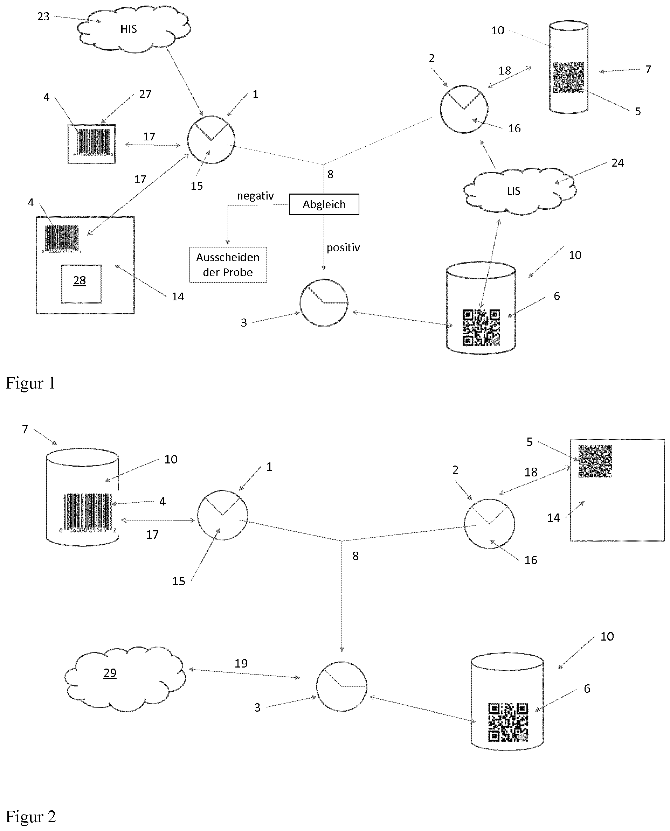

Die Figuren und die dazugehörende Figurenbeschreibung sind keinesfalls auf den Gegenstand der hier offenbarten Erfindung einschränkend zu werten. Der Fachmann ist zweifelslos in der Lage, die in den Figuren gezeigten Ausführungsformen des Verfahrens miteinander zu kombinieren. Ebenso ist der Fachmann in der Lage, die gezeigte Vorrichtung so anzupassen, dass die in Figur 1 und 2 gezeigten Verfahren effizient durchgeführt werden können.

Figur 1 veranschaulicht eine Ausführungsform des erfindungsgemäßen Verfahrens, welche Ausführungsform innerhalb eines Krankenhauses anwendbar ist.

Die ersten Daten 1 sind mit einem ersten Code 4 über eine erste Datenverknüpfung 17 verknüpft, welcher erster Code 4 auf einem Erkennungsband 27 und auf einem Registerblatt 14 angeordnet ist. Die Figur 1 ist in dieser Hinsicht eine vereinfachte Darstellung, da das Erkennungsband 27 und das Registrierblatt 14 einen identen ersten Code 4 aufweisen. Es ist durch die Offenbarung der Erfindung nicht ausgeschlossen, dass das Erkennungsband 27 und das Registerblatt 14 unterschiedliche erste Codes aufweisen.

Das Registerblatt 14 kann neben dem ersten Code 4 auch weitere niedergeschriebene Kontrolldaten 28 wie Namen und Geburtsdatum des Patienten umfassen, die auch in den ersten Daten 1 enthalten sind. Die auf dem Registerblatt 14 angeführten Kontrolldaten 28 können auch Anweisungen an den Benutzer in Schriftform umfassen, welche auch als erste Daten 1 in der HIS-Datenbank 23 abgespeichert sind.

Durch Lesen des ersten Codes 4 können erste Daten 1 von einer HIS-Datenbank 23 abgerufen werden. Die HIS-Datenbank 23 ist eine Cloud-Datenbank.

Figur 1 zeigt weiters einen Behälter 10 mitsamt einer darin gelagerten Probe. Der Behälter 10 und die Probe bilden das Objekt 7, welches Objekt 7 durch einen zweiten Code 5 gekennzeichnet ist. Der zweite Code 5 wird während der Produktion des Behälters 10 auf den Behälter 10 aufgedruckt. Der zweite Code 5 ist somit mit dem Behälter 10 untrennbar verbunden.

Der zweite Code 5 ist über eine zweite Datenverknüpfung 18 mit zweiten Daten 2 verknüpft, welche durch Lesen des zweiten Codes 5 von einer LIS-Datenbank 24 abrufbar sind. Die LIS-Datenbank kann eine vom Hersteller des Behälters 10 betriebene Cloud-Datenbank sein.

Vor der Abnahme einer Probe von einer Person scannt der Benutzer den am Erkennungsband angeordneten ersten Code 4 und den am Registerblatt 14 angeordneten ersten Code 4. Hierdurch werden von der HIS-Datenbank 23 erste Daten 1 heruntergeladen, welche erste Daten 1 im Wesentlichen Angaben über die Person und eine Beschreibung des Auftrages zur Durchführung der Abnahme der Probe betreffen.

Der Benutzer liest weiters den auf dem Behälter 10 angebrachten zweiten Code 5 ab, wodurch von der LIS-Datenbank 24 zweite Daten 2 heruntergeladen werden, welche zweiten Daten 2 im Wesentlichen den Behälter 10 betreffen.

In einem zeitlich nachfolgenden Verfahrensschritt werden die ersten Teilbereiche 15 der ersten Attribute der ersten Daten 1 mit zweiten Teilbereichen 16 der zweiten Attribute der zweiten Daten 2 zusammengeführt.

Die Auswahl der ersten Teilbereiche 15 aus den ersten Daten 1 erfolgt in Abhängigkeit der durchzuführenden Analysen an der Probe. Im Wesentlichen werden jene ersten Attribute aus den ersten Daten 1 ausgewählt, welche zur Durchführung der Analyse der Probe notwendig sind und welche zur Durchführung der unten beschriebenen Abfrageroutinen notwendig sind.

Die Auswahl der zweiten Teilbereiche 16 aus den zweiten Daten 2 erfolgt ebenso in Abhängigkeit der durchzuführenden Analysen an der Probe. Es werden auch hier jene zweiten Attribute aus den zweiten Daten 2 ausgewählt, welche zweiten Attribute zur Durchführung der Analyse der Probe notwendig sind und welche zur Durchführung der unten beschriebenen Abfrageroutinen erforderlich sind.

Die Auswahl der ersten Teilbereiche 15 und der zweiten Teilbereiche 16 erfolgt sohin nach medizinischen Aspekten, sodass Analyseverfahren nach dem Stand der Technik an der Probe durchgeführt werden können, und nach dem unten erläuterten Abgleich von Attributen.

Der Verfahrensschritt des Zusammenführens von ersten Daten 1 und zweiten Daten 2 wird in Abhängigkeit eines Abgleiches von zugeordneten passenden Attributen der ersten Daten 1 und der zweiten Daten 2 durchgeführt. Figur 1 umfasst bei der symbolisierten Zusammenführung 8 der ersten Daten 1 und der zweiten Daten 2 eine Schaltfläche "Abgleich". Ein positiver Abgleich führt zu der Generierung der dritten Daten 3. Ein negativer Abgleich für zu einem Ausscheiden der Probe.

Es können als Teil des in Figur 1 dargestellten Verfahrens die Eigenschaften des Behälters 10 abgeglichen werden. Die ersten Daten 1 umfassen ein erstes Attribut, dass die Probe in einen Behälter 10 bestimmten Eigenschaften erfüllen muss. Gemäß dieser Anforderung an den Behälter 10 muss dieser ein bestimmtes Additiv aufweisen, welches sich mit der Probe vermengt. Die zweiten Daten 2 umfassen ein zweites Attribut, dass der Behälter 10 diese Eigenschaften aufweist. Das erste Attribut und das zweite Attribut können als zueinander passend (positiver Abgleich) angesehen werden, da der Benutzer den richtigen Behälter 10 aus einer in Figur 10 nicht dargestellten Behältermenge ausgewählt hat. Das Zusammenführen der ersten Daten 1 und der zweiten Daten 2 kann in Hinblick auf diese Abfrage durchgeführt werden.

Falls der Benutzer nicht den richtigen Behälter 10 ausgewählt hat und sohin das erste Attribut und das zweite Attribut als nicht passend (negativer Abgleich) angesehen werden, so wird das Objekt 7 ausgeschieden. Ergänzend erhält der Benutzer eine Aufforderung zum nochmaligen Einbringen einer Probe in einen weiteren Behälter.

Es kann weiters als ein Teil des in Figur 1 dargestellten Verfahrens das Alter des Behälters 10 angesehen werden. Die zweiten Daten 1 umfassen weiters ein Attribut, dass der Behälter 10 mitsamt dem darin zur Vermengung mit der Probe gelagerten Attribut nicht älter als ein gewisses Datum sein darf. Das zweite Attribut gilt als passend zu dem ersten Attribut, wenn das zweite Attribut die durch das erste Attribut definierte Anforderung erfüllt. Das Zusammenführen der ersten Daten 1 und der zweiten Daten 2 kann sohin in Hinblick hierauf durchgeführt werden.

Die ersten Teilbereiche 15 der ersten Attribute der ersten Daten 1 und die zweiten Teilbereiche 16 der zweiten Attribute der zweiten Daten 2 werden zu dritten Daten 3 zusammengeführt. Die dritten Daten 3 werden mit einem dritten Code 6 verknüpft und in der LIS-Datenbank 24 abgespeichert. Durch Lesen des dritten Codes 6 sind die dritten Daten 3 von der LIS-Datenbank 24 abrufbar.

Die dritten Daten 3 umfassen lediglich Attribute, welche zur Durchführung der Analyse der Probe notwendig sind. Durch Lesen des dritten Codes 6 können nicht jene ersten Attribute der ersten Daten 1 abgerufen werden, welche nicht in der ersten Teilmenge 15 enthalten waren.

Figur 2 veranschaulicht eine Ausführungsform des erfindungsgemäßen Verfahrens, welche Ausführungsform beispielsweise im Gesundheitswesen Anwendung finden kann.

Figur 2 zeigt einen Behälter 10 als ein erstes Objekt 7, in welchem Behälter 10 eine Flüssigkeit als Probe gelagert ist. Die als Probe im Behälter 10 eingelagerte Flüssigkeit kann beispielsweise eine Blutprobe, eine Urinprobe oder ähnliches sein. Es ist auf der Manteloberfläche des Behälters 10 zur Kennzeichnung des ersten Objektes 7 ein Barcode als erster Code 4 aufgebracht.

Der erste Code 4 ist über eine erste Datenverknüpfung 17 mit einer Menge von ersten Daten 1 verknüpft, welche ersten Daten 1 in einer HIS-Datenbank (in Figur 1 nicht dargestellt) abgespeichert sind. Derartige Systeme zum Verknüpfen von Daten, hier von ersten Daten 1, mit einem Code, hier mit dem ersten Code 4, sind nach dem Stand der Technik zu der Offenbarung dieser Erfindung bekannt.

Die ersten Daten 1 beschreiben im Wesentlichen das Objekt 7, die im Behälter 10 gelagerte Probe, zeitliche Angaben über die Herstellung der Probe und die Einbringung dieser in den Behälter 10.

Die ersten Daten 1 beschreibend das Objekt 7 können beispielsweise erste Attribute über das Material der Objektes 7, ein im Behälter 10 gelagertes Additiv zum Vermengen mit der einzubringenden Probe, ein zeitliches Ablaufdatum einer Verwendung der Probe et cetera umfassen.

Die ersten Daten 1 können weitere erste Attribute beschreibend die Probe wie beispielsweise die Art der Probe, Angaben über den Patienten, Zeitpunkt der Entnahme der Probe, Zeitpunkt der Einbringung der Probe in den Behälter 10 et cetera umfassen.

Die ersten Daten 1 können weiters eine Angabe über die zeitliche Verwendung der Probe für Analysen umfassen. Diese können automatisch auf Basis der oben genannten zeitlichen Angaben erstellt werden.

Die durch die ersten Attribute gebildete Menge an ersten Daten 1 ist in Figur 2 durch ein Tortendiagramm dargestellt. Die ersten Daten 1 stellen sogenannte krankenhausbezogene Daten dar. Üblicher Weise werden die ersten Daten 1 im Krankenhaus gesammelt und ein erster Code 4 erstellt, welcher erster Code 4 nur durch im Krankenhaus installierte Lesegeräte und nicht durch im Labor installierte Lesegeräte auslesbar ist.

Figur 2 zeigt weiters ein Registerblatt 14, welches üblicher Weise in Labors Anwendung findet. Das Registerblatt 14 umfasst beispielsweise Angaben, wann welche Analysemethoden an der Probe durchgeführt werden sollen. Diese Angaben sind über eine zweite Datenverknüpfung 18 mit einem QR-Code als zweiten Code 5 verknüpft. Die zweiten Daten 2 sind durch Lesen des zweiten Codes 5 aus einer LIS-Datenbank (in Figur 2 nicht dargestellt) abrufbar. Diese Angaben bilden die zweiten Attribute der zweiten Daten 2, welche wiederum als ein Tortendiagramm in Figur 2 dargestellt sind.

Labor verwenden in der Regel ein anderes System als Krankenhäuser. Aus diesem Grund ist der erste Code 4 durch die im Labor installierten Lesegeräte und der zweite Code 5 durch die im Krankenhaus installierten Lesegeräte nicht auslesbar. In Figur 2 ist die übliche Verwendung von unterschiedlichen Codes durch einen Barcode als ersten Code 4 und einen QR-Code als zweiten Code 5 veranschaulicht.

Bei Anwendung des erfindungsgemäßen Verfahrens werden die ersten Daten 1 durch Lesen des ersten Codes 4 von der HIS-Datenbank und die zweiten Daten 2 durch Lesen des zweiten Codes 5 von der LIS-Datenbank abgerufen. Das Lesen der Codes erfolgt durch einen Computer mitsamt einem geeigneten Auslesegerät, welcher Computer sowohl den ersten Code 4 als auch den zweiten Code 5 auslesen kann. Der Computer ist weiters mit der HIS-Datenbank und der LIS-Datenbank verbunden.

Aus einer ersten Teilmenge 15 der ersten Attribute der ersten Daten 1 und aus einer zweiten Teilmenge 16 der zweiten Attribute der zweiten Daten 2 wird eine Menge dritter Daten 3 durch Zusammenführen 8 der ersten Daten 1 und der zweiten Daten 2 generiert. Die Attribute der dritten Daten 3 bestehen aus ersten Attributen und zweiten Attributen.

Die erste Teilmenge 15 der ersten Daten 1 wird durch die ersten Attribute gebildet, welche ersten Attribute unbedingt für die Durchführung der Analysen und zur Identifizierung der Probe erforderlich sind. Die Bildung der ersten Teilmenge 15 kann in Bezugnahme auf die zweiten Attribute unmittelbar nach dem Auslesen der zweiten Daten automatisch erfolgen.

Die in der Teilmenge 15 nicht enthaltenden ersten Attribute wie persönliche Angaben zum Patienten werden nicht in die dritten Daten 3 aufgenommen. Hierdurch wird verhindert, dass diese persönlichen Angaben in der LIS-Datenbank abgespeichert und von dort unbefugt ausgelesen werden.

Die erstellten dritten Daten 3 werden mit einem dritten Code 6 über eine dritte Datenverknüpfung 19 mit einer dritten Datenbank 25 verknüpft. Der dritte Code 6 ist derart gestaltet, dass dieser von im Labor installierten Lesegeräten auslesbar ist. Durch die Generierung des dritten Codes 6 wird die erste Teilmenge 15 von ersten Attributen für die Laborgeräte lesbar gemacht. Die erste Teilmenge 15 von ersten Attributen wird auf eine äußerst effiziente Weise unter Berücksichtigung eines notwendigen vertraulichen Umgangs mit sensiblen Daten im Gesundheitsbereich in der dritte Datenbank 29 abgespeichert.

Der dritte Code 6 wird über den ursprünglich am Behälter 10 angebrachten ersten Code 4 am Behälter 10 angebracht. Der erste Code 4, welcher durch den dritten Code 6 verdeckt ist, ist bei der Untersuchung im Labor nicht mehr sichtbar und sohin nicht mehr auslesbar.

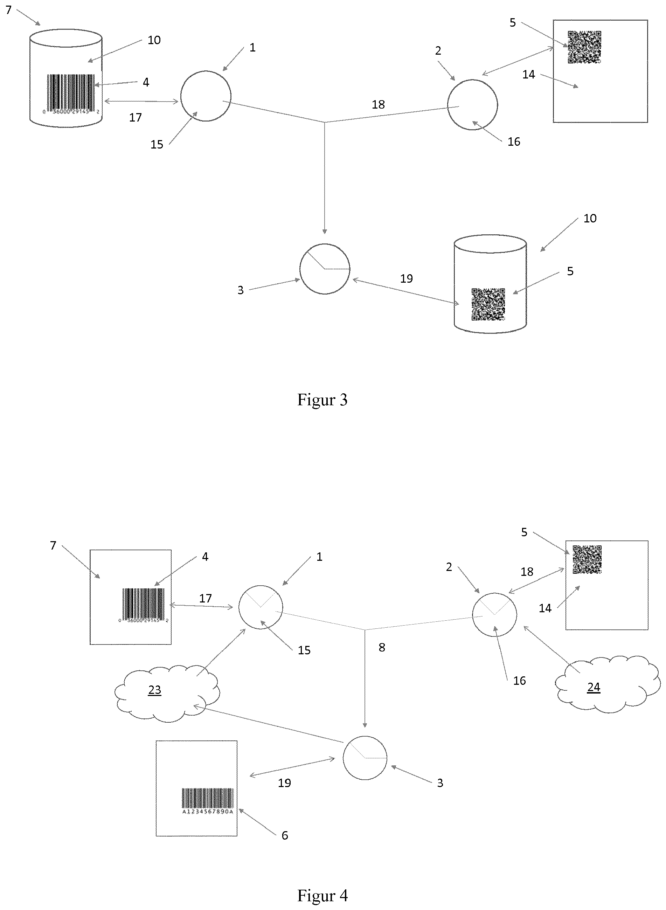

Figur 3 veranschaulicht eine weitere Ausführungsform des erfindungsgemäßen Verfahrens. Ein eine Probe beinhaltender Behälter 10, an dessen Oberfläche ein erster Code 4 aufgebracht ist, bildet ein Objekt 7. Der erste Code 4 ist über die Benützung einer Datenbank erstellt worden. Der erste Code 4 ist über eine erste Datenverknüpfung mit den ersten Daten 1 verknüpft, wobei die ersten Daten 1 in der Datenbank (in Figur 3 nicht dargestellt) abgespeichert sind.

Ein in einem Labor üblicher Weise verwendetes Registerblatt 14 umfasst einen zweiten Code 5, welcher zweiter Code 5 über eine zweite Datenverknüpfung mit den zweiten Daten 2 verknüpft ist. Die zweiten Daten 2 umfassen zweite Attribute. Die zweiten Daten 2 werden über ein Lesen des zweiten Codes 5 von der Datenbank abgerufen.

Der erste Code 4 und der zweite Code 5 sind nicht für alle Geräte auslesbar. Einige Geräte können nur den ersten Code 4 oder den zweiten Code 5 lesen.

In einem Verfahrensschritt wird eine zweite Teilmenge 16 von zweiten Attributen der zweiten Daten 2 ausgelesen, welche mit einem zweiten Code 5 verknüpft und in der Datenbank abgespeichert sind. Die zweiten Daten 2 werden über ein Lesen des zweiten Codes 5 von der Datenbank abgerufen.

Parallel hierzu wird durch Lesen des ersten Codes 4 eine erste Teilmenge 15 von ersten Attributen der ersten Daten 1 von der Datenbank abgerufen. Weder Figur 2 noch die Beschreibung zu Figur 2 sind auf eine bestimmte zeitliche Reihenfolge des Lesens des ersten Codes 4 und des zweiten Codes 5 beschränkt.

Bei dem mittels Figur 3 veranschaulichten Verfahren umfassen die ausgewählte erste Teilmenge 15 die gesamten ersten Attribute der ersten Daten 1 und die zweite Teilmenge 16 die gesamten zweiten Attribute der zweiten Daten 2.

Die in der Datenbank abgespeicherten ersten Daten 1 und zweiten Daten 2 werden zu dritten Daten 3 zusammengeführt, welche wiederum in der Datenbank abgespeichert werden und mit einem dritten Code 6 über eine dritte Datenverknüpfung 19 verknüpft sind.

Der dritte Code 6 ist auf dem Behälter 10 so positioniert, dass der dritte Code 6 den ersten Code 4 überdeckt.

Figur 4 zeigt eine weitere Anwendungsmöglichkeit des erfindungsgemäßen Verfahrens. Durch Lesen des ersten Codes 4, welcher auf einem Objekt 7 aufgedruckt ist, sind erste Daten 1 von einer HIS-Datenbank 23 unter Aktivierung und Benutzung einer ersten Datenverbindung 17 abrufbar. Das Objekt 7 ist eine Karteikarte, welche im Krankenhaus verwendet wird.

In hierzu analoger Weise sind durch Lesen des auf einem Registerblatt 14 angeordneten zweiten Code 5 zweite Daten 2 von einer LIS-Datenbank 24 unter Aktivierung und Verwendung einer zweiten Datenverbindung 18 abrufbar. Das Registerblatt 14 umfasst die Ergebnisse einer zuvor durchgeführten Untersuchung im Labor. Die Ergebnisse der Untersuchung sind auch in Form einer zweiten Teilmenge 16 der zweiten Daten 2 in der LIS-Datenbank 24 abgespeichert. Die zweiten Daten 2 können neben den Ergebnissen der Untersuchung auch weitere Daten wie Daten über die Belegung der Laborgeräte umfassen, welche für das Krankenhaus nicht von Interesse sind.

Beim Zusammenführen der ersten Daten 1 und der zweiten Daten 2 wird die zweite Teilmenge 16 ausgewählt. Dies ist mittels Verfahren nach dem Stand der Technik möglich, da die zweite Teilmenge 16 die Ergebnisse der Untersuchung umfasst.

Die Anwendung des erfindungsgemäßen Verfahrens löst das Problem der sicheren Zuordnung der Laborergebnisse zu einem Patienten beziehungsweise zu einem Untersuchungsauftrag, welcher in der HIS-Datenbank 23 abgespeichert ist.

Es wird von den ersten Daten 1 eine erste Teilmenge 15 abgerufen. Die erste Teilmenge 15 kann die Auftragsdaten zur Durchführung der Laboruntersuchungen umfassen. Die ersten Daten 1 können neben der ersten Teilmenge 15 auch persönliche Patientendaten umfassen, welche aus Datenschutzgründen für den anschließend vorgenommenen Abgleich der ersten Teilmenge 15 und der zweiten Teilmenge 16 nicht von der HIS-Datenbank 23 abgerufen werden.

Beim Zusammenführen der ersten Teilmenge 15 und der zweiten Teilmenge 16 wird der in der ersten Teilmenge 15 enthaltene Untersuchungsauftrag und die in der zweiten Teilmenge 16 enthaltenen Ergebnisse der Untersuchung abgeglichen und hieraus dritten Daten 3 generiert.

Die dritten Daten 6 werden unter Aktivierung einer dritten Datenverbindung 19 in der HIS-Datenbank 23 gespeichert. Es wird weiters ein dritter Code 6 erstellt, welcher auf das Objekt 7 den ersten Code 4 überdeckend erstellt, welcher dritter Code 6 mit den dritten Daten 3 über eine dritte Datenverknüpfung 19 verbunden ist. Hierdurch wird sichergestellt, dass nur Benutzer die Möglichkeit des Abrufens der die Ergebnisse der Untersuchung umfassenden dritten Daten 3 haben, welche den dritten Code 6 haben.

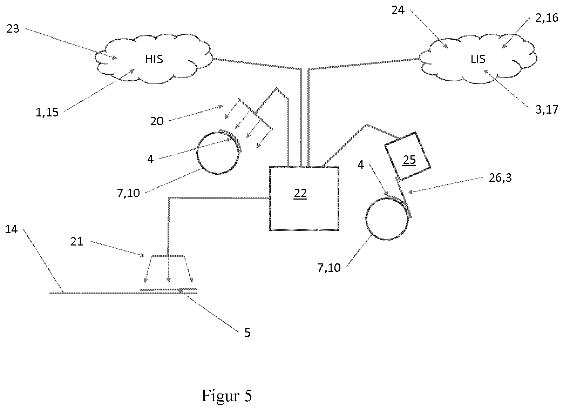

Figur 5 veranschaulicht die Elemente einer Vorrichtung zur Durchführung des erfindungsgemäßen Verfahrens.

Die Vorrichtung umfasst eine erste Leseeinheit 20 zum Auslesen des ersten Codes 4. Die erste Leseeinheit 20 erlaubt das kontaktlose Lesen des ersten Codes 4. Derartige Leseeinheiten sind nach dem Stand der Technik bekannt.

Die Vorrichtung umfasst eine zweite Leseeinheit 21 zum Lesen des zweiten Codes 5.

Die Codegenerierungseinheit zum Generieren des dritten Codes 6 ist in einer Recheneinheit 22 integriert. Die Recheneinheit 22 ist über Kabel oder auch hierzu gleichwertig über Funk mit der HIS-Datenbank 23 und mit der LIS-Datenbank 24 verbunden. Über die Recheneinheit 22 besteht ein Zugriff auf die in der HIS-Datenbank 23 abgespeicherten ersten Daten 1 und die in der LIS-Datenbank abgespeicherten zweiten Daten 2.

Die Vorrichtung umfasst weiters einen Drucker 25, mittels welchem Drucker 25 der dritte Code 6 auf eine Folie 26 ausgedruckt wird. Der Drucker 25 umfasst weiters eine Einheit zum Positionieren der Folie 26 mitsamt dem dritten Code 6 über dem ersten Code 4.

Die Recheneinheit 22 erstellt dritte Daten 3 aus einem ersten Teilbereich 15 der ersten Attribute der ersten Daten 1 und einem zweiten Teilbereich 16 der zweiten Attribute der zweiten Daten 2. Die dritten Daten 3 werden in der LIS-Datenbank abgespeichert und mit dem dritten Code 6 verknüpft.

Während und/oder nach einer Untersuchung der im Behälter 10 gelagerten Probe werden die Untersuchungsdaten in der LIS -Datenbank 24 als vierte Daten gespeichert. Die Untersuchungsdaten sind Messwerte und Messergebnisse, welche durch eine Untersuchung der Probe erstellt werden. Die vierten Daten umfassen die dritten Daten 3 und die Untersuchungsdaten.

Die Recheneinheit 22 übermittelt die vierten Daten oder die Untersuchungsdaten an die HIS-Datenbank 23.

### Bezugszeichenaufstellung

- 1: erste Daten
- 2: zweite Daten
- 3: dritte Daten
- 4: erster Code
- 5: zweiter Code
- 6: dritter Code
- 7: Objekt
- 8: (frei)
- 9: (frei)
- 10: Behälter
- 11: (frei)
- 12: (frei)
- 13: (frei)
- 14: Registerblatt
- 15: erste Teilmenge
- 16: zweite Teilmenge
- 17: erste Datenverbindung
- 18: zweite Datenverbindung
- 19: dritte Datenverbindung
- 20: erste Leseeinheit
- 21: zweite Leseeinheit
- 22: Recheneinheit
- 23: HIS-Datenbank
- 24: LIS-Datenbank
- 25: Drucker
- 26: Folie
- 27: Erkennungsband
- 28: Kontrolldaten
- 29: dritte Datenbank

## Patentansprüche

1. Verfahren zur Zusammenführung von ersten Daten (1) und zweiten Daten (2), welche Daten (1,2) erste Attribute beziehungsweise zweite Attribute umfassen und welche Daten über einen ersten Code (4) beziehungsweise über einen zweiten Code (5) auslesbar sind, umfassend die folgenden Verfahrensschritte:
- Abrufen der ersten Daten (1),
- Abrufen der zweiten Daten (2),
- Zusammenführen der ersten Daten (1) und der zweiten Daten (2),
- Generierung von dritten Daten (3) umfassend eine erste Teilmenge (15) der ersten Attribute und eine zweite Teilmenge (16) der zweiten Attribute,
- Generierung eines dritten Codes (6) zum Auslesen der dritten Daten,
- Überschreiben des ersten Codes (4) mit dem dritten Code (6).

2. Verfahren nach Anspruch 1, **dadurch gekennzeichnet, dass**
der zweite Code (5) und der dritte Code (6) ident sind.

3. Verfahren nach einem der Ansprüche 1 bis 2, **dadurch gekennzeichnet, dass**
der Verfahrensschritt des Zusammenführens von ersten Daten (1) und zweiten Daten (6) in Abhängigkeit von passenden Attributen der ersten Daten (1) und der zweiten Daten (2) durchgeführt wird.

4. Verfahren nach Anspruch 3, **dadurch gekennzeichnet, dass**
ein mit den ersten Daten (1) verknüpftes erstes Objekt ausgeschieden wird, wenn die Anzahl der passenden Attribute kleiner als ein definierter Schwellenwert ist.

5. Verfahren nach einem der Ansprüche 1 bis 4, **dadurch gekennzeichnet, dass**
die dritten Daten (3) einen ersten Zeitpunkt des Auslesens der ersten Daten umfassen.

6. Verfahren nach Anspruch 5, **dadurch gekennzeichnet, dass**
ein mit den ersten Daten (1) verknüpftes Objekt (7) ausgeschieden wird, wenn der erste Zeitpunkt nach einem definierten Auslesezeitpunkt liegt.

7. Vorrichtung zur Durchführung des Verfahrens nach einem der vorangegangenen Ansprüche, **dadurch gekennzeichnet, dass**
die Vorrichtung
eine erste Datenausleseeinheit (20) zum Auslesen der ersten Daten,
eine zweite Datenausleseeinheit (21) zum Auslesen der zweiten Daten,
eine Codegenerierungseinheit zum Generieren des dritten Codes (6) und
eine Überschreibeinheit zum Überschreiben des ersten Codes (4) durch den dritten Code (6) umfasst.

8. Vorrichtung nach Anspruch 7, **dadurch gekennzeichnet, dass**
die Codegenerierungseinheit einen Drucker (25) umfasst, mittels welchem Drucker (25) der dritte Code (6) auf eine Folie (26) ausgedruckt wird, und
die Überschreibeinheit die Folie (26) den ersten Code (4) verdeckend am Objekt (7) anbringt.
